# EUROPEAN PATENT SPECIFICATION

(11) **EP 3 563 832 B1**
(45) Date of publication and mention of the grant of the patent: **09.09.2026**
(21) Application number: 19178833.0
(22) Date of filing: 02.12.2016
(51) Int. Cl.: A61K 31/58, A61K 31/167, A61K 9/08, A61K 47/12

(54) **PHARMACEUTICAL COMPOSITION**
PHARMAZEUTISCHE ZUSAMMENSETZUNG
COMPOSITION PHARMACEUTIQUE

(30) Priority: 04.12.2015 GB 201521462
(43) Date of publication of application: 06.11.2019
(62) Document number(s) of the earlier application(s) in accordance with Art. 76 EPC: 16823027.4 / 3 383 367
(73) Proprietor: Mexichem Fluor S.A. de C.V., 78395 San Luis Potosi S.L.P. (MX)
(72) Inventor: Corr, Stuart, Warrington, Cheshire WA4 5DH (GB); Noakes, Timothy James, Flintshire, CH7 5JF (GB)
(74) Representative: Potter Clarkson

(56) References cited:
- WO-A1-03/086350
- WO-A1-2008/097233
- WO-A2-03/020253
- WO-A2-2014/016548
- US-B1- 6 432 415

## Description

The present invention relates to the delivery of drug formulations from a medical device, such as a metered dose inhaler (MDI), using a propellant comprising 1,1-difluoroethane (HFA-152a). More particularly, the present invention relates to pharmaceutical compositions comprising R-152a propellant and a binary drug formulation which is dissolved or suspended in the propellant and to medical devices containing those compositions. The pharmaceutical compositions of the invention are particularly suited for delivery from a pressurised aerosol container using a metered dose inhaler (MDI).

MDIs are the most significant type of inhalation drug delivery system and are well known to those skilled in the art. They are designed to deliver, on demand, a discrete and accurate amount of a drug to the respiratory tract of a patient using a liquefied propellant in which the drug is dissolved, suspended or dispersed. The design and operation of MDIs is described in many standard textbooks and in the patent literature. They all comprise a pressurised container that holds the drug formulation, a nozzle and a valve assembly that is capable of dispensing a controlled quantity of the drug through the nozzle when it is activated. The nozzle and valve assembly are typically located in a housing that is equipped with a mouth piece. The drug formulation will comprise a propellant, in which the drug is dissolved, suspended or dispersed, and may contain other materials such as polar excipients, surfactants and preservatives.

In order for a propellant to function satisfactorily in MDIs, it needs to have a number of properties. These include an appropriate boiling point and vapour pressure so that it can be liquefied in a closed container at room temperature but develop a high enough pressure when the MDI is activated to deliver the drug as an atomised formulation even at low ambient temperatures. Further, the propellant should be of low acute and chronic toxicity and have a high cardiac sensitisation threshold. It should have a high degree of chemical stability in contact with the drug, the container and the metallic and non-metallic components of the MDI device, and have a low propensity to extract low molecular weight substances from any elastomeric materials in the MDI device. The propellant should also be capable of maintaining the drug in a homogeneous solution, in a stable suspension or in a stable dispersion for a sufficient time to permit reproducible delivery of the drug in use. When the drug is in suspension in the propellant, the density of the liquid propellant is desirably similar to that of the solid drug in order to avoid rapid sinking or floating of the drug particles in the liquid. Finally, the propellant should not present a significant flammability risk to the patient in use. In particular, it should form a non-flammable or low flammability mixture when mixed with air in the respiratory tract.

Dichlorodifluoromethane (R-12) possesses a suitable combination of properties and was for many years the most widely used MDI propellant, often blended with trichlorofluoromethane (R-11). Due to international concern that fully and partially halogenated chlorofluorocarbons (CFCs), such as dichlorodifluoromethane and trichlorofluoromethane, were damaging the earth's protective ozone layer, many countries entered into an agreement, the Montreal Protocol, stipulating that their manufacture and use should be severely restricted and eventually phased out completely. Dichlorodifluoromethane and trichlorofluoromethane were phased out for refrigeration use in the 1990's, but are still used in small quantities in the MDI sector as a result of an essential use exemption in the Montreal Protocol.

1,1,1,2-tetrafluoroethane (R-134a) was introduced as a replacement refrigerant and MDI propellant for R-12. 1,1,1,2,3,3,3-heptafluoropropane (R-227ea) was also introduced as a replacement propellant for dichlorotetrafluoroethane (R-114) in the MDI sector and is sometimes used alone or blended with R-134a for this application.

Although R-134a and R-227ea have low ozone depletion potentials (ODPs), they have global warming potentials (GWPs), 1430 and 3220 respectively, which are now considered to be too high by some regulatory bodies, especially for dispersive uses when they are released into the atmosphere.

One industrial area that has received particular attention recently has been the automotive air-conditioning sector where the use of R-134a has come under regulatory control as a result of the European Mobile Air Conditioning Directive (2006/40/EC). Industry is developing a number of possible alternatives to R-134a in automotive air conditioning and other applications that have a low greenhouse warming potential (GWP) as well as a low ozone depletion potential (ODP). Many of these alternatives include hydrofluoropropenes, especially the tetrafluoropropenes, such as 2,3,3,3-tetrafluoropropene (R-1234yf) and 1,3,3,3-tetrafluoropropene (R-1234ze).

Although the proposed alternatives to R-134a have a low GWP, the toxicological status of many of the components, such as certain of the fluoropropenes, is unclear and they are unlikely to be acceptable for use in the MDI sector for many years, if at all.

There are also other problems with R-134a and R-227ea. Most pharmaceutical actives for treating respiratory disorders, such as asthma, tend not to dissolve well in either R-134a or R-227ea and have to be handled as suspensions in the propellant. Drug suspensions give rise to a number of problems, such as nozzle blockage, agglomeration and sedimentation, the latter problem making it essential to shake the MDI thoroughly before use to ensure that the drug is evenly distributed in the propellant. Furthermore, if the pharmaceutical active settles quickly following re-suspension in the propellant, as is often the case, then the propellant/drug composition must be delivered from the MDI shortly after shaking in order to ensure that the dose that is delivered contains an effective concentration of the pharmaceutical active.

The problem of poorly dissolving drugs has been addressed by including a polar excipient in the composition which either helps to dissolve the drug to form a solution or else enhances wetting of suspended drug particles to yield a better dispersed and more stable suspension. A preferred polar excipient is ethanol. However, the use of large amounts of ethanol can tend to result in a coarse spray having droplet sizes that are too large for acceptable penetration into the deep bronchiole passages of the lung. Further, high levels of ethanol can have unacceptable irritancy to the mouth and throat, especially with younger users and may be unacceptable on religious grounds.

Surfactants have also been included in some formulations that include drugs that are either insoluble or only sparingly soluble in the propellant, as these can also help to produce a more stable suspension. However, surfactants must be selected carefully for acceptability in the lung and add an additional layer of formulation complexity. Accordingly, it would be beneficial to form a stable suspension without the use of a surfactant.

A commonly used drug for treating asthma and chronic obstructive pulmonary disease (COPD) is formoterol, most commonly in the form of its dihydrate fumarate salt. Formoterol is a selective, long-acting β₂-adrenergic agonist (LABA) that can be delivered to the respiratory tract using a MDI. Unfortunately, it has proven difficult to formulate formoterol in a form that is suitable for delivery using MDI technology due to its limited physical and chemical stability. The problem of stability is particularly evident when the formoterol is exposed to other components that are often used in pharmaceutical formulations, including excipients, solvents, e.g. ethanol, and other therapeutic agents. Other therapeutic agents that are used in combination with formoterol include corticosteroids and more particularly the glucocorticosteroids. Particularly desirable combination formulations include formoterol with one or more corticosteroids selected from mometasone (often as the furoate), budesonide, beclomethasone (often as the dipropionate) and fluticasone (often as the propionate).

The instability of pharmaceutical formulations of formoterol can result in a limited shelf life at ambient temperatures and can necessitate refrigerated storage prior to use.

WO 03/020253 discloses a pharmaceutical aerosol formulation comprising mometasone furoate, formoterol fumarate and either 1,1,1,2,3,3,3-heptafluoropropane (HFA-227ea) or 1,1,1,2-tetrafluoroethane (HFA-134a) as the propellant. 1,1-difluoroethane (HFA-152a) is not disclosed.

WO 2014/016548 discloses a pharmaceutical composition comprising a bronchodilator and a corticosteroid. Mometasone is mentioned as one possible corticosteroid. The composition may be in a form suitable for administration using a dry powder inhaler or it may be in a form suitable for delivery using a metered dose inhaler when a propellant is incorporated in the composition. Suitable propellants are said to include 1,1,1,2,3,3,3-heptafluoropropane (HFA-227ea), 1,1,1,2-tetrafluoroethane (HFA-134a) and 1,1-difluoroethane (HFA-152a).

WO 2008/097233 discloses a pharmaceutical composition comprising an active pharmaceutical agent and optionally a propellant. Mometasone furoate is mentioned as an active pharmaceutical agent and 1,1,1,2,3,3,3-heptafluoropropane (HFA-227ea) and 1,1,1,2-tetrafluoroethane (HFA-134a) are mentioned as propellants. 1,1-difluoroethane (HFA-152a) is not disclosed.

WO 03/086350 discloses a pharmaceutical aerosol formulation comprising a formoterol compound, a mometasone compound and either 1,1,1,2,3,3,3-heptafluoropropane (HFA-227ea) or 1,1,1,2-tetrafluoroethane (HFA-134a) as the propellant. 1,1-difluoroethane (HFA-152a) is not disclosed.

US 6,432,415 discloses a bio-adhesive formulation comprising 1,1-difluoroethane (HFA-152a). Mometasone is not disclosed.

There is a need for a pharmaceutical composition of mometasone and formoterol, which can be delivered using a MDI and that uses a propellant having a reduced GWP in comparison with R-134a and R-227ea. There is also a need for a pharmaceutical composition of mometasone and formoterol, which exhibits improved storage stability.

According to a first aspect of the present invention there is provided a pharmaceutical composition, especially a dispersion, comprising:
(i) at least one mometasone compound selected from mometasone and mometasone furoate;
(ii) formoterol fumarate dihydrate; and
(iii) a propellant component at least 90 weight % of which is 1,1-difluoroethane (R-152a).

The pharmaceutical composition of the first aspect of the invention typically contains less than 500 ppm of water based on the total weight of the pharmaceutical composition. In a preferred embodiment, the pharmaceutical composition of the first aspect of the invention contains less than 100 ppm, preferably less than 50 ppm, more preferably less than 10 ppm and particularly less than 5 ppm of water based on the total weight of the pharmaceutical composition. In referring to the water content of the pharmaceutical composition, we are referring to the content of free water in the composition and not any water that happens to be present in any hydrated drug compounds that may be used as part of the drug component. In an especially preferred embodiment, the pharmaceutical composition is water-free. Alternatively, the pharmaceutical composition of the first aspect may contain greater than 0.5 ppm of water, e.g. 1 ppm or greater, but less than the amounts discussed above, as it can in practice be difficult to remove all the water from the composition and then retain it in such a water-free state. Low water contents are preferred because they tend to reduce the degradation of the drug compounds resulting in a composition with higher chemical stability.

Accordingly, a preferred embodiment of the first aspect of the present invention provides a pharmaceutical composition, especially a dispersion, said composition comprising:
(i) at least one mometasone compound selected from mometasone and mometasone furoate;
(ii) formoterol fumarate dihydrate; and
(iii) a propellant component at least 90 weight % of which is 1,1-difluoroethane (R-152a),
wherein the composition contains less than 100 ppm, preferably less than 50 ppm, more preferably less than 10 ppm and particularly less than 5 ppm of water based on the total weight of the pharmaceutical composition.

In a preferred embodiment, the pharmaceutical composition of the first aspect of the invention contains less than 1000 ppm, preferably less than 500 ppm, more preferably less than 100 ppm and particularly less than 50 ppm of dissolved oxygen based on the total weight of the pharmaceutical composition. In an especially preferred embodiment, the pharmaceutical composition is oxygen-free. Alternatively, the pharmaceutical composition of the first aspect may contain greater than 0.5 ppm of oxygen, e.g. 1 ppm or greater, but less than the amounts discussed above, as it can in practice be difficult to retain the composition in an oxygen-free state. Low oxygen contents are preferred because they tend to reduce the degradation of the drug compounds resulting in a composition with higher chemical stability.

Accordingly, a preferred embodiment of the first aspect of the present invention provides a pharmaceutical composition, especially a dispersion, said composition comprising:
(i) at least one mometasone compound selected from mometasone and mometasone furoate;
(ii) formoterol fumarate dihydrate; and
(iii) a propellant component at least 90 weight % of which is 1,1-difluoroethane (R-152a),
wherein the composition contains less than 1000 ppm, preferably less than 500 ppm, more preferably less than 100 ppm and particularly less than 50 ppm of oxygen based on the total weight of the pharmaceutical composition.

In one embodiment, the pharmaceutical composition of the first aspect of the present invention consists essentially of the three components (i) to (iii) listed above. In another embodiment, the pharmaceutical composition of the first aspect of the present invention consists entirely of the three components (i) to (iii) listed above. By the term "consists essentially of", we mean that at least 95 weight %, more preferably at least 98 weight % and especially at least 99 weight % of the pharmaceutical composition consists of the three listed components.

The pharmaceutical composition of the first aspect of the present invention is suitable for delivery to the respiratory tract using a metered dose inhaler (MDI).

In a preferred embodiment, the pharmaceutical composition of the first aspect of the present invention is free of acid stabilisers, such as organic and inorganic acids.

The pharmaceutical composition of the first aspect of the present invention may additionally include a polar excipient, such as ethanol. Polar excipients are used routinely in pharmaceutical compositions for treating respiratory disorders that are delivered using metered dose inhalers (MDIs). They are also referred to as solvents, co-solvents, carrier solvents and adjuvants. Their inclusion can serve to solubilise a surfactant or the drug in the propellant and/or inhibit deposition of drug particles on the surfaces of the metered dose inhaler that are contacted by the pharmaceutical composition as it passes from the container in which it is stored to the nozzle outlet. They are also used as bulking agents in two-stage filling processes where the drug is mixed with a suitable polar excipient. The most commonly used polar excipient is ethanol. If a polar excipient is used, it will typically be present in an amount of from 0.5 to 10 % by weight, preferably in an amount of from 1 to 5 % by weight based on the total weight of the pharmaceutical composition.

In one embodiment, the pharmaceutical composition of the first aspect of the present invention is free of polar excipients such as ethanol.

The pharmaceutical composition of the first aspect of the present invention may additionally include a surfactant component comprising at least one surfactant compound.

Surfactant compounds of the type that have been in use hitherto in pharmaceutical formulations for MDIs may be used in the pharmaceutical compositions of the present invention. Preferred surfactants are selected from polyvinylpyrrolidone, polyethylene glycol surfactants, oleic acid and lecithin. If a surfactant component is included, it will preferably consist essentially of and still more preferably consist entirely of at least one surfactant compound selected from polyvinylpyrrolidone, polyethylene glycols, oleic acid and lecithin. Oleic acid is especially preferred. By oleic acid, we are referring, in particular, to the commercially available surfactant material which may not be 100 % pure. By the term "consists essentially of", we mean that at least 95 weight %, more preferably at least 98 weight % and especially at least 99 weight % of the surfactant component is composed of at least one of the listed surfactants.

In one embodiment, the pharmaceutical composition of the first aspect of the present invention is surfactant free.

The at least one mometasone compound and the formoterol fumarate dihydrate in the pharmaceutical composition of the invention in all aspects and embodiments disclosed herein are preferably in a micronized form. Further, the pharmaceutical composition of the invention in all aspects and embodiments disclosed herein is preferably free of perforated microstructures.

The at least one mometasone compound and the formoterol fumarate dihydrate may be dispersed or suspended in the propellant. The drug particles in such dispersions/suspensions preferably have a diameter of less than 100 microns, e.g. less than 50 microns. The pharmaceutical composition of the invention may also be a solution with the at least one mometasone compound and the formoterol fumarate dihydrate dissolved in the propellant, e.g. with the assistance of a polar excipient, such as ethanol. Preferably, the pharmaceutical composition of the invention is a dispersion.

In a preferred embodiment, the at least one mometasone compound comprises mometasone furoate. Especially preferred pharmaceutical compositions of the invention are those in which the at least one mometasone compound consists essentially of mometasone furoate. By the term "consists essentially of", we mean that at least 95 weight %, more preferably at least 98 weight % and especially at least 99 weight % of the at least one mometasone compound is mometasone furoate. Most preferred pharmaceutical compositions of the invention are those in which the at least one mometasone compound is entirely mometasone furoate.

In a particularly preferred embodiment, the pharmaceutical composition of the first aspect of the present invention comprises both mometasone furoate and formoterol fumarate dihydrate. Preferably, mometasone furoate and formoterol fumarate dihydrate are the only pharmaceutical actives in the pharmaceutical composition of the first aspect of the present invention.

The weight ratio of the at least one mometasone compound, e.g. mometasone furoate, to the formoterol fumarate dihydrate is typically in the range of from 100:1 to 10:1, preferably in the range of from 40:1 to 20:1.

The propellant component in the pharmaceutical composition of the present invention comprises 1,1-difluoroethane (R-152a). Thus, we do not exclude the possibility that the propellant component may include other propellant compounds in addition to the R-152a. For example, the propellant component may additionally comprise one or more additional hydrofluorocarbon or hydrocarbon propellant compounds, e.g. selected from R-227ea, R-134a, difluoromethane (R-32), propane, butane, isobutane and dimethyl ether. The preferred additional propellants are R-227ea and R-134a.

If an additional propellant compound is included, such as R-134a or R-227ea, the R-152a will constitute at least 90 weight %, e.g. from 90 to 99 weight %, of the propellant component. Preferably, the R-152a will constitute at least 95 weight %, e.g. from 95 to 99 weight %, and more preferably at least 99 weight % of the propellant component.

In an especially preferred embodiment, the propellant component consists entirely of HFA-152a so that the pharmaceutical composition of the invention comprises HFA-152a as the sole propellant. By the term "consists entirely of" we do not, of course, exclude the presence of minor amounts, e.g. up to a few hundred parts per million, of impurities that may be present following the process that is used to make the HFA-152a providing that they do not affect the suitability of the propellant in medical applications. Preferably the HFA-152a propellant will contain no more than 10 ppm, e.g. from 0.5 to 10 ppm, more preferably no more than 5 ppm, e.g. from 1 to 5 ppm, of unsaturated impurities, such as vinyl fluoride, vinyl chloride, vinylidene fluoride and chloro-fluoro ethylene compounds.

It will be apparent from the discussion above that in a preferred embodiment of the present invention, there is provided a pharmaceutical composition comprising:
(i) mometasone furoate;
(ii) formoterol fumarate dihydrate; and
(iii) a propellant component at least 90 weight % of which is 1,1-difluoroethane (R-152a).

In this preferred embodiment, the pharmaceutical composition preferably consists essentially of and more preferably is composed entirely of the three listed components (i) to (iii). By the term "consists essentially of", we mean that at least 95 weight %, more preferably at least 98 weight % and especially at least 99 weight % of the pharmaceutical composition consists of the three listed components (i) to (iii). In addition, the propellant component preferably consists essentially of and more preferably consists entirely of 1,1-difluoroethane (R-152a). By the term "consists essentially of", we mean that at least 95 weight %, more preferably at least 98 weight % and especially at least 99 weight % of the propellant component consists of R-152a. Furthermore, the amounts of water and oxygen in the pharmaceutical composition of this preferred embodiment are as discussed above.

The pharmaceutical composition of the first aspect of the present invention typically comprises from 0.01 to 1.0 weight % of the at least one mometasone compound and the formoterol fumarate dihydrate combined and from 99.0 to 99.99 weight % of the propellant component. Preferred compositions comprise from 0.05 to 0.5 weight % of the at least one mometasone compound and the formoterol fumarate dihydrate combined and from 99.5 to 99.95 weight % of the propellant component. Particularly preferred pharmaceutical compositions comprise from 0.07 to 0.3 weight % of the at least one mometasone compound and the formoterol fumarate dihydrate combined and from 99.7 to 99.93 weight % of the propellant component. All percentages are based on the total weight of the pharmaceutical compositions.

It has been found that the use of propellants comprising 1,1-difluoroethane (R-152a) in pharmaceutical compositions containing a mometasone compound, such as mometasone furoate, either alone or together with a formoterol compound, such as formoterol fumarate dihydrate, can unexpectedly improve the chemical stability of the mometasone and formoterol compounds compared to the stability they exhibit in known formulations containing either R-134a or R-227ea as the propellant.

Accordingly, also described herein but not claimed is a method of improving the stability of a pharmaceutical composition comprising a propellant component and at least one mometasone compound selected from mometasone and its pharmaceutically acceptable derivatives, such as pharmaceutically acceptable salts of mometasone, prodrugs of mometasone, solvates of mometasone, solvates of pharmaceutically acceptable salts of mometasone and solvates of prodrugs of mometasone which is dissolved or suspended in the propellant component, said method comprising using a propellant component comprising 1,1-difluoroethane (R-152a).

The improved chemical stability can result, in particular, when the pharmaceutical composition contains less than 500 ppm, preferably less than 100 ppm, more preferably less than 50 ppm, still more preferably less than 10 ppm and particularly less than 5 ppm of water based on the total weight of the pharmaceutical composition. In referring to the water content of the pharmaceutical composition, we are referring to the content of free water in the composition and not any water that happens to be present in any hydrated drug compounds that may be used. In an especially preferred embodiment, the pharmaceutical composition is water-free. Alternatively, the pharmaceutical composition that is used in the stabilisation method may contain greater than 0.5 ppm of water, e.g. greater than 1 ppm, but less than the amounts discussed above, as it can in practice be difficult to remove all the water from the composition and then retain it in such a water-free state.

In practice, preparing a pharmaceutical composition with the low water levels recited above involves using a propellant component with a suitably low water content, as it is usually the largest mass item in the finished device, and then preparing the pharmaceutical composition under suitably dry conditions, e.g. in a dry nitrogen atmosphere. Preparing pharmaceutical compositions under dry conditions is well known and the techniques involved are well understood by those skilled in the art. Other steps to obtain a low water content in the finished device include drying and storing the can and valve components in a moisture-controlled atmosphere, e.g. dry nitrogen or air, prior to and during device assembly. If the pharmaceutical composition contains a significant amount of ethanol, then it may also be important to control the water content of the ethanol as well as the propellant, e.g. by drying to reduce the water content to suitably low levels. Suitable drying techniques are well known to those skilled in the art and include the use of a molecular sieve or other inorganic desiccant and membrane drying processes.

In the stabilisation method, the preferred mometasone compound is mometasone furoate. In addition, typical and preferred amounts of the mometasone compound and the propellant component in the stabilisation method and suitable, typical and preferred compositions for the propellant component are as discussed above for the pharmaceutical composition of the first aspect of the invention.

In preferred stabilisation methods described herein, the pharmaceutical composition additionally comprises at least one formoterol compound selected from formoterol and its pharmaceutically acceptable derivatives, such as pharmaceutically acceptable salts of formoterol, prodrugs of formoterol, solvates of formoterol, solvates of pharmaceutically acceptable salts of formoterol and solvates of prodrugs of formoterol. When a formoterol compound is included, the preferred formoterol compound is formoterol fumarate dihydrate.

In one stabilisation method, the pharmaceutical composition consists essentially of and more preferably consists entirely of the drug component(s) and the propellant component as defined above. By the term "consists essentially of", we mean that at least 95 weight %, preferably at least 98 weight %, more preferably at least 99 weight % and especially at least 99.9 weight % of the pharmaceutical composition consists of the two components.

The pharmaceutical composition that is used in the stabilisation method may also contain one or both of a polar excipient and a surfactant component as discussed above for the pharmaceutical composition of the first aspect of the invention. Suitable and preferred polar excipients and surfactants are as discussed above for the pharmaceutical composition of the first aspect of the invention. Typical and preferred amounts of the polar excipient and the surfactant component are as discussed above for the pharmaceutical composition of the first aspect of the invention.

In one preferred stabilisation method in which the pharmaceutical composition also comprises at least one formoterol compound, the resulting pharmaceutical composition after storage, e.g. in a coated or uncoated aluminium container, at 40°C and 75 % relative humidity for 3 months will produce less than 1.0 % by weight, preferably less than 0.8 % by weight, more preferably less than 0.7 % by weight and still more preferably less than 0.6 % by weight of impurities from the degradation of the at least one mometasone compound and the at least one formoterol compound based on the total weight of the at least one mometasone compound, the at least one formoterol compound and the impurities.

In another preferred stabilisation method in which the pharmaceutical composition also comprises at least one formoterol compound, the resulting pharmaceutical composition after storage, e.g. in a coated or uncoated aluminium container, at 25°C and 60 % relative humidity for 3 months will produce less than 1.0 % by weight, preferably less than 0.8 % by weight, more preferably less than 0.7 % by weight and still more preferably less than 0.5 % by weight of impurities from the degradation of the at least one mometasone compound and the at least one formoterol compound based on the total weight of the at least one mometasone compound, the at least one formoterol compound and the impurities.

In still another preferred stabilisation method in which the pharmaceutical composition also comprises at least one formoterol compound, at least 97.0 % by weight and preferably at least 97.5 % by weight of the at least one mometasone compound and the at least one formoterol compound that are contained originally in the pharmaceutical composition immediately following preparation will be present in the composition after storage, e.g. in a coated or uncoated aluminium container, at 40°C and 75 % relative humidity for 3 months.

In a further preferred stabilisation method, at least 97.0 % and preferably at least 97.5 % of the original pharmaceutical activity of the composition is retained after storage, e.g. in a coated or uncoated aluminium container, at 40°C and 75 % relative humidity for 3 months.

The improved stability that is observed in accordance with the stabilisation method described herein is attainable for compositions that contain a surfactant, such as oleic acid, as well as those that are surfactant-free.

In a particularly preferred stabilisation method, the pharmaceutical composition that is provided in the method is free of acid stabilisers, such as organic and inorganic acids.

Accordingly, a preferred pharmaceutical composition of the first aspect of the present invention is one that produces less than 1.0 % by weight, preferably less than 0.8 % by weight, more preferably less than 0.7 % by weight and still more preferably less than 0.6 % by weight of total impurities from the degradation of the pharmaceutical actives, i.e. the at least one mometasone compound and the formoterol fumarate dihydrate, after storage, e.g. in a coated or uncoated aluminium container, at 40°C and 75 % relative humidity for 3 months.

A further preferred pharmaceutical composition of the first aspect of the present invention is one that produces less than 1.0 % by weight, preferably less than 0.8 % by weight, more preferably less than 0.7 % by weight and still more preferably less than 0.5 % by weight of total impurities from the degradation of the pharmaceutical actives, i.e. the at least one mometasone compound and the formoterol fumarate dihydrate, after storage, e.g. in a coated or uncoated aluminium container, at 25°C and 60 % relative humidity for 3 months.

The weight % of impurities indicated above are based on the total weight of the at least one mometasone compound, the at least one formoterol compound (when included) and the impurities.

In a further preferred pharmaceutical composition of the first aspect of the present invention at least 97.0 % by weight and preferably at least 97.5 % by weight of the at least one mometasone compound and the formoterol fumarate dihydrate that are contained originally in the pharmaceutical composition immediately following preparation will be present in the composition after storage, e.g. in a coated or uncoated aluminium container, at 40°C and 75 % relative humidity for 3 months.

In yet another preferred pharmaceutical composition of the first aspect of the present invention at least 97.0 % and preferably at least 97.5 % of the original pharmaceutical activity of the pharmaceutical composition is retained after storage, e.g. in a coated or uncoated aluminium container, at 40°C and 75 % relative humidity for 3 months.

The improved stability that is observed for the pharmaceutical compositions of the invention is attainable for compositions that contain a surfactant, such as oleic acid, as well as those that are surfactant-free. In addition, it is attainable for compositions that are free of acid stabilisers, such as organic and inorganic acids.

In referring to the storage of the pharmaceutical compositions in the above described stabilisation methods, we are referring, in particular, to the storage of those compositions in coated or uncoated aluminium containers. Similarly, in referring to the storage of the above described pharmaceutical compositions, we are referring, in particular, to their storage in coated or uncoated aluminium containers.

It has been found that the use of a propellant comprising 1,1-difluoroethane (HFA-152a) in surfactant-free pharmaceutical compositions containing a mometasone compound, such as mometasone furoate, a formoterol compound, such as formoterol fumarate dihydrate, and the propellant that are designed to be delivered using a metered dose inhaler can unexpectedly improve the aerosolization performance of the pharmaceutical composition when that composition is delivered from the metered dose inhaler compared to the performance that is observed when either HFA-134a or HFA-227ea is used as the propellant. In particular, the fine particle fractions of both the mometasone and formoterol compounds in the emitted dose typically comprise at least 30 weight % and preferably at least 35 weight % of the emitted dose of the mometasone and formoterol compounds. We are referring here, in particular, to the emitted dose that is observed immediately after the pharmaceutical composition has been filled into a MDI canister and prior to any long term storage.

Accordingly, also described herein but not claimed is a method of improving the aerosolization performance of a surfactant-free pharmaceutical composition comprising a propellant component and a drug component comprising at least one mometasone compound and at least one formoterol compound as defined herein, said method comprising using a propellant component comprising 1,1-difluoroethane (HFA-152a).

The pharmaceutical composition in the method of improving the aerosolization performance described herein may be a suspension or a solution, but is typically a suspension.

A preferred method of improving the aerosolization performance provides a pharmaceutical composition which when delivered from a metered dose inhaler yields fine particle fractions of both the at least one mometasone compound and the at least one formoterol compound which are at least 30 weight % and preferably at least 35 weight % of the emitted doses of the at least one mometasone compound and the at least one formoterol compound. We are referring here, in particular, to the emitted dose that is observed immediately after the pharmaceutical composition has been filled into a MDI canister and prior to any long term storage.

Increasing the fine particle fraction of the emitted dose is highly beneficial, because it is the fine drug particles that are able to penetrate into the deep bronchiole passages and the alveolar passages of the lung to maximise relief from the effects of an asthma attack or COPD.

The fine particle fraction is a widely recognised term in the art. It is a measure of the mass fraction of emitted aerosol particles having a diameter below 5 µm which is generally accepted as being the most desirable particle size range for effective alveolar drug delivery.

Suitable and preferred mometasone compounds and suitable and preferred formoterol compounds in the method of improving the aerosolization performance described herein are as described above for the pharmaceutical composition of the first aspect of the present invention. In addition, typical and preferred amounts of the drug components and the propellant component in the method of improving the aerosolization performance and suitable, typical and preferred compositions for the propellant component are as discussed above for the pharmaceutical composition of the first aspect of the invention.

In one method of improving the aerosolization performance, the pharmaceutical composition consists essentially of and more preferably consists entirely of the drug components and the propellant component as defined above. By the term "consists essentially of", we mean that at least 95 weight %, preferably at least 98 weight %, more preferably at least 99 weight % and especially at least 99.9 weight % of the pharmaceutical composition consists of the two components.

The pharmaceutical composition in the method of improving the aerosolization performance described herein may also contain a polar excipient as discussed above for the pharmaceutical composition of the first aspect of the invention. Suitable and preferred polar excipients are as discussed above for the pharmaceutical composition of the first aspect of the invention. Typical and preferred amounts of the polar excipient are as discussed above for the pharmaceutical composition of the first aspect of the invention.

In a particularly preferred method of improving the aerosolization performance, the pharmaceutical composition that is provided in the method is free of acid stabilisers, such as organic and inorganic acids.

It has also been found that the use of a propellant comprising 1,1-difluoroethane (HFA-152a) in pharmaceutical compositions containing a mometasone compound, such as mometasone furoate, a formoterol compound, such as formoterol fumarate dihydrate, and the propellant that are designed to be delivered using a metered dose inhaler can unexpectedly improve the aerosolization performance of the pharmaceutical composition after storage when that composition is delivered from the metered dose inhaler compared to the performance that is observed when either HFA-134a or HFA-227ea is used as the propellant.

Accordingly, also described herein but not claimed is a method of improving the aerosolization performance after storage, e.g. in coated or uncoated aluminium containers, of a pharmaceutical composition comprising a propellant component and a drug component comprising at least one mometasone compound as defined herein and at least one formoterol compound as defined herein, said method comprising using a propellant component comprising 1,1-difluoroethane (HFA-152a).

The pharmaceutical composition in the method of improving the aerosolization performance after storage may be a suspension or a solution, but is typically a suspension.

Increasing the fine particle fraction of the emitted dose after long term storage is highly beneficial. As explained above, it is the fine drug particles that are able to penetrate into the deep bronchiole passages and the alveolar passages of the lung to maximise relief from the effects of an asthma attack or COPD. Thus, retaining a high fine particle fraction after storage means that the user of the MDI should still receive a medically satisfactory dose of the drug even though a significant period of time has elapsed since the pharmaceutical composition was first manufactured.

In the method of improving the aerosolization performance after storage described herein suitable and preferred mometasone compounds and suitable and preferred formoterol compounds are as described above for the pharmaceutical composition of the first aspect of the present invention. In addition, typical and preferred amounts of the drugs and the propellant component in the method of improving the aerosolization performance after storage described herein and suitable, typical and preferred compositions for the propellant component are as discussed above for the pharmaceutical composition of the first aspect of the invention.

In one method of improving the aerosolization performance after storage described herein, the pharmaceutical composition consists essentially of and more preferably consists entirely of the drugs and the propellant component as defined above. By the term "consists essentially of", we mean that at least 95 weight %, preferably at least 98 weight %, more preferably at least 99 weight % and especially at least 99.9 weight % of the pharmaceutical composition consists of the two components.

The pharmaceutical composition in the method of improving the aerosolization performance after storage described herein may also contain one or both of a polar excipient and a surfactant component as discussed above for the pharmaceutical composition of the first aspect of the invention. Suitable and preferred polar excipients and surfactants are as discussed above for the pharmaceutical composition of the first aspect of the invention. Typical and preferred amounts of the polar excipient and the surfactant component are as discussed above for the pharmaceutical composition of the first aspect of the invention.

In a particularly preferred method of improving the aerosolization performance after storage, the pharmaceutical composition that is provided in the method is free of acid stabilisers, such as organic and inorganic acids.

It has been found that the use of propellants comprising 1,1-difluoroethane (HFA-152a) in pharmaceutical compositions containing a mometasone compound as defined herein, optionally together with a formoterol compound as defined herein, that is dispersed or suspended in the propellant can unexpectedly increase the time it takes for the particulate drug(s) to settle following thorough dispersion in the propellant compared to the settling times that are observed when either HFA-134a or HFA-227ea is used as the propellant.

Accordingly, also described herein but not claimed is a method of increasing the settling time of a pharmaceutical composition comprising a propellant component and a drug component suspended in the propellant component comprising a mometasone compound as defined herein, optionally together with a formoterol compound as defined herein, said method comprising using a propellant component comprising 1,1-difluoroethane (HFA-152a).

In one preferred method of increasing the settling time as described herein, the settling time is at least 2.0 minutes following complete dispersion in the HFA-152a-containing propellant, more preferably at least 2.5 minutes, still more preferably at least 3.0 minutes.

Suitable and preferred mometasone compounds and suitable and preferred formoterol compounds in the method of increasing the settling time as described herein are as described above for the pharmaceutical composition of the first aspect of the present invention. In addition, typical and preferred amounts of the drug(s) and the propellant component in the method of increasing the settling time as described herein and suitable, typical and preferred compositions for the propellant component are as discussed above for the pharmaceutical composition of the first aspect of the invention.

In one method of increasing the settling time, the pharmaceutical composition consists essentially of and more preferably consists entirely of the drug(s) and the propellant component as defined above. By the term "consists essentially of", we mean that at least 95 weight %, preferably at least 98 weight %, more preferably at least 99 weight % and especially at least 99.9 weight % of the pharmaceutical composition consists of the two components.

The pharmaceutical composition in the method of increasing the settling time described herein may also contain one or both of a polar excipient and a surfactant component as discussed above for the pharmaceutical composition of the first aspect of the invention. Suitable and preferred polar excipients and surfactants are as discussed above for the pharmaceutical composition of the first aspect of the invention. Typical and preferred amounts of the polar excipient and the surfactant component are as discussed above for the pharmaceutical composition of the first aspect of the invention.

In a particularly preferred method of increasing the settling time, the pharmaceutical composition that is provided in the method is free of acid stabilisers, such as organic and inorganic acids.

The pharmaceutical compositions of the invention find particular utility in the delivery of the mometasone and formoterol compounds from pressurised aerosol containers, e.g. using a metered dose inhaler (MDI). For this application, the pharmaceutical compositions are contained in the pressurised aerosol containers and the R-152a propellant functions to deliver the drug as a fine aerosol spray.

The pharmaceutical compositions of the invention may comprise one or more other additives of the type that are conventionally used in drug formulations for pressurised MDIs, such as valve lubricants. Where other additives are included in the pharmaceutical compositions, they are normally used in amounts that are conventional in the art.

The pharmaceutical compositions of the invention are normally stored in pressurised containers or canisters which are to be used in association with a medication delivery device. When so stored, the pharmaceutical compositions are normally a liquid. In a preferred embodiment, the pressurised container is designed for use in a metered dose inhaler (MDI). In a particularly preferred embodiment, the pressurised container is a coated aluminium can or an uncoated aluminium can, especially the latter given its lower cost and ease of use.

Accordingly, a second aspect of the present invention provides a pressurised container holding a pharmaceutical composition of the first aspect of the present invention. In a third aspect, the present invention provides a medication delivery device, especially a metered dose inhaler, having a pressurised container holding the pharmaceutical composition of the first aspect of the present invention.

The metered dose inhaler typically comprises a nozzle and valve assembly that is crimped to a container holding the pharmaceutical composition to be dispensed. An elastomeric gasket is used to provide a seal between the container and the nozzle/valve assembly. Preferred elastomeric gasket materials are EPDM, chlorobutyl, bromobutyl and cycloolefin copolymer rubbers as these can exhibit good compatibility with HFA-152a and also provide a good barrier to prevent or limit HFA-152a permeating from the container.

The pharmaceutical compositions of the present invention are suitable for use in medicine for treating a patient suffering or likely to suffer from a respiratory disorder and especially asthma or a chronic obstructive pulmonary disease.

Accordingly, also described herein but not claimed is a method for treating a patient suffering or likely to suffer from a respiratory disorder, especially asthma or a chronic obstructive pulmonary disease, which comprises administering to the patient a therapeutically or prophylactically effective amount of a pharmaceutical composition of the first aspect of the invention. The pharmaceutical compositions are preferably delivered to the patient using a MDI.

The pharmaceutical compositions of the invention can be prepared by a simple blending operation in which the at least one mometasone compound, the formoterol fumarate dihydrate, the R-152a-containing propellant and any other optional components are mixed together in the required proportions in a suitable mixing vessel. Mixing can be promoted by stirring as is common in the art. Conveniently, the R-152a-containing propellant is liquefied to aid mixing. If the pharmaceutical composition is made in a separate mixing vessel, it can then be transferred to pressurised containers for storage, such as pressurised containers that are used as part of medication delivery devices and especially MDIs.

The pharmaceutical compositions of the invention can also be prepared within the confines of a pressurised container, such as an aerosol canister or vial, from which the compositions are ultimately released as an aerosol spray using a medication delivery device, such as a MDI. In this method, a weighed amount of the at least one mometasone compound and the formoterol fumarate dihydrate are introduced into the open container. A valve is then crimped onto the container and the R-152a-containing propellant component, in liquid form, introduced through the valve into the container under pressure, optionally after first evacuating the container through the valve. A surfactant component and any other optional components can be mixed with the mometasone or, alternatively, introduced into the container after the valve has been fitted, either alone or as a premix with the propellant component. The whole mixture can then be treated to disperse the drugs in the mixture, e.g. by vigorous shaking or using an ultrasonic bath. Suitable containers may be made of plastics, metal, e.g. aluminium, or glass. Preferred containers are made of metal, especially aluminium which may be coated or uncoated.

Uncoated aluminium containers are especially preferred as they are cheaper and easier to use.

The container may be filled with enough of the pharmaceutical composition to provide for a plurality of dosages. The pressurized aerosol canisters that are used in MDIs typically contain 50 to 150 individual dosages.

The present invention is now illustrated by the following examples.

### Example 1

A number of experiments were conducted to investigate the *in vitro* aerosolization performance of pharmaceutical formulations of mometasone furoate and formoterol fumarate dihydrate delivered from a metered dose inhaler (MDI) using either HFA-227ea or HFA-152a as the propellant after initial preparation and after storing under stress storage conditions.

Pharmaceutical formulations of micronized mometasone furoate (0.17 % w/w), micronized formoterol fumarate dihydrate (0.01 % w/w), ethanol (2.70 % w/w) and optionally oleic acid (1.70 % w/w) were prepared in either HFA-227ea or HFA-152a (Mexichem, UK) with the propellant making up the balance. The oleic acid (if included) was first mixed with the ethanol. The drugs were weighed directly into a glass vessel to which the ethanol or ethanol/oleic acid mixture was added. This mixture was then homogenized using a Silverson LS5 mixer at 4000 rpm for 20 minutes. The slurry was then dispensed into 14 ml coated aluminium canisters and stainless steel canisters. The canisters were then crimped with a 50 µL valve (Aptar, France) following which the propellant was filled into the canisters through the valve using a manual Pamasol crimper/filler (Pamasol, Switzerland). Finally, the canisters were sonicated for 20 minutes to aid dispersion of the drugs in the suspension.

The *in vitro* aerosolization performance of the formulations was tested immediately after preparation (time t = zero) with a Next Generation Impactor using the method described below. The formulations were then stored under stress storage conditions (valve down) at 40°C and 75 % relative humidity for 1 month and 3 months. After storing for 1 month and 3 months under the stress storage conditions, the *in vitro* aerosolization performance of the pharmaceutical formulations was tested again as before with a Next Generation Impactor using the method described below.

The Next Generation Impactor (NGI, Copley Scientific, Nottingham UK) was connected to a vacuum pump (GE Motors, NJ, USA). Prior to testing, the cups of the NGI system were coated with 1 % v/v silicone oil in hexane to eliminate particle bounce. For each experiment, three actuations of the valve were discharged into the NGI at 30 L.min⁻¹ as per pharmacopeia guidelines. Following aerosolization, the NGI apparatus was dismantled and the actuator and each part of the NGI was washed down into known volumes of the HPLC mobile phase. The mass of drug deposited on each part of the NGI was determined by HPLC. This protocol was repeated three times for each canister, following which, the fine particle dose (FPD) and fine particle fraction of the emitted dose (FPF_{ED}) were determined.

High performance liquid chromatography (HPLC) was used to determine drug content following the aerosolization studies. A 100 mm x 3.0 mm Accucore Phenyl-X column with a 2.6 µm particle size was used for the analysis. The column was maintained at 40°C and was coupled to a UV detector operating at a wavelength of 250 nm. The autosampler was operated at ambient temperature and 100 µl samples were injected into the column for the analyses. The run time was 27 minutes and the flow rate 0.55 ml/minute. The chromatographic conditions are shown in Tables 1 and 2 below.

**Table 1**

| **Drug** | **Pump Flow Rate (ml.min⁻¹)** | **Mobile Phase (gradient elution)** | **UV Wavelength (nm)** | **Column Temperature (°C)** |
|---|---|---|---|---|
| Mometasone furoate and formoterol fumarate dihydrate | 0.55 | Mobile Phase A: Aqueous ammonium formate solution adjusted to pH 3.0 with formic acid | 250 | 40 |
| | | Mobile Phase B: Acetonitrile | | |

The composition of the mobile phase was varied as shown in Table 2 below.

**Table 2**

| **Time (minutes)** | **Volume % Mobile Phase A** | **Volume % Mobile Phase B** |
|---|---|---|
| 0.0 | 90 | 10 |
| 16.0 | 0 | 100 |
| 20.0 | 0 | 100 |
| 20.1 | 90 | 10 |
| 25.0 | 90 | 10 |

The results are shown in Tables 3A, 3B, 4A, 4B, 5A and 5B below.

**Table 3A. In vitro aerosolization performance of mometasone emitted from MDI combination formulations of mometasone and formoterol in HFA-227ea and HFA-152a with and without oleic acid as characterised by the emitted dose, fine particle dose, mass median aerodynamic diameter (MMAD) and geometric standard deviation (GSD).**

| | **HFA 227ea** | **HFA 227ea (No Oleic)** | **HFA 152a** | **HFA 152a (No Oleic)** |
|---|---|---|---|---|
| Emitted Dose/µg | 97.8 (0.5) | 93.0 (4.5) | 99.1 (0.8) | 98.5 (1.9) |
| Fine Particle Dose/ µg | 33.8 (0.9) | 26.1 (0.5) | 37.5 (1.8) | 35.6 (0.7) |
| MMAD ± GSD | 3.28 (1.82) | 3.50 (1.91) | 3.37 (1.83) | 3.32 (1.82) |

**Table 3B. In vitro aerosolization performance of formoterol emitted from MDI combination formulations of mometasone and formoterol in HFA-227ea and HFA-152a with and without oleic acid as characterised by the emitted dose, fine particle dose, mass median aerodynamic diameter (MMAD) and geometric standard deviation (GSD).**

| | **HFA 227ea** | **HFA 227ea (No Oleic)** | **HFA 152a** | **HFA 152a (No Oleic)** |
|---|---|---|---|---|
| Emitted Dose/µg | 4.8 (0.1) | 4.7 (0.2) | 4.7 (0.1) | 4.6 (0.2) |
| Fine Particle Dose/ µg | 1.9 (0.2) | 1.3 (0.1) | 1.9 (0.1) | 1.9 (0.1) |
| MMAD ± GSD | 3.14 (2.04) | 3.43 (2.11) | 3.21 (2.00) | 3.17 (1.99) |

**Table 4A. In vitro aerosolization performance of mometasone emitted from MDI combination formulations of mometasone and formoterol in HFA-227ea and HFA-152a with oleic acid as characterised by the emitted dose, fine particle dose, fine particle fraction of the emitted dose (FPF_{ED}), mass median aerodynamic diameter (MMAD) and geometric standard deviation (GSD). These data are at Tzero, T = 1 Month @ 40°C/75 % RH valve down and T = 3 Months @ 40°C/75 % RH valve down.**

| | **HFA 227 - Tzero** | **HFA 227 - 1M@40/75** | **HFA 227 - 3M@40/75** | **152a - Tzero** | **152a - 1M@40/75** | **152a - 3M@40/75** |
|---|---|---|---|---|---|---|
| Emitted Dose (µg) | 97.8 ± 0.5 | 95.1 ± 0.5 | 91.6 ± 0.2 | 99.1 ± 0.8 | 95.8 ± 0.5 | 99.8 ± 0.9 |
| Fine Particle Dose (µg) | 33.8 ± 0.9 | 34.2 ± 0.3 | 27.7 ± 0.5 | 37.5 ± 1.8 | 39.0 ± 1.5 | 32.5 ± 1.1 |
| FPF_{ED} (%) | 34.6 | 36.0 | 30.2 | 37.8 | 40.7 | 32.6 |
| MMAD (µm) | 3.28 | 3.33 | 3.68 | 3.37 | 3.42 | 3.49 |
| GSD | 1.82 | 1.82 | 1.76 | 1.83 | 1.82 | 1.81 |

**Table 4B. In vitro aerosolization performance of mometasone emitted from MDI combination formulations of mometasone and formoterol in HFA-227ea and HFA-152a without oleic acid as characterised by the emitted dose, fine particle dose, fine particle fraction of the emitted dose (FPF_{ED}), mass median aerodynamic diameter (MMAD) and geometric standard deviation (GSD). These data are at Tzero, T = 1 Month @ 40°C/75 % RH valve down and T =3 Months @ 40°C/75 % RH valve down.**

| | **HFA 227 - Tzero** | **HFA 227 - 1M@40/75** | **HFA 227 - 3M@40/75** | **152a - Tzero** | **152a - 1M@40/75** | **152a - 3M@40/75** |
|---|---|---|---|---|---|---|
| Emitted Dose (µg) | 93.0 ± 4.5 | 89.3 ± 0.7 | 89.5 ± 0.7 | 98.5 ± 1.9 | 98.6 ± 0.9 | 98.8 ± 1.1 |
| Fine Particle Dose (µg) | 26.1 ± 0.5 | 21.4 ± 0.7 | 18.4 ± 0.8 | 35.6 ± 0.7 | 34.7 ± 1.9 | 32.5 ± 1.9 |
| FPF_{ED} (%) | 28.1 | 24.0 | 20.6 | 36.1 | 35.2 | 32.9 |
| MMAD (µm) | 3.50 | 3.78 | 3.78 | 3.32 | 3.26 | 3.49 |
| GSD | 1.91 | 1.86 | 1.89 | 1.82 | 1.86 | 1.82 |

**Table 5A. In vitro aerosolization performance of formoterol emitted from MDI combination formulations of mometasone and formoterol in HFA-227ea and HFA-152a with oleic acid as characterised by the emitted dose, fine particle dose, fine particle fraction of the emitted dose (FPF_{ED}), mass median aerodynamic diameter (MMAD) and geometric standard deviation (GSD). These data are at Tzero, T = 1 Month @ 40°C/75 % RH valve down and T = 3 Months @ 40°C/75 % RH valve down.**

| | **HFA 227 - Tzero** | **HFA 227 - 1M@40/75** | **HFA 227 - 3M@40/75** | **152a - Tzero** | **152a - 1M@40/75** | **152a - 3M@40/75** |
|---|---|---|---|---|---|---|
| Emitted Dose (µg) | 4.8 ± 0.1 | 4.1 ± 0.2 | 4.9 ± 0.3 | 4.7 ± 0.1 | 4.8 ± 0.2 | 4.9 ± 0.1 |
| Fine Particle Dose (µg) | 1.9 ± 0.2 | 1.6 ± 0.1 | 1.4 ± 0.1 | 1.9 ± 0.1 | 2.0 ± 0.1 | 2.1 ± 0.2 |
| FPF_{ED} (%) | 39.6 | 39.0 | 28.6 | 40.4 | 41.7 | 42.9 |
| MMAD (µm) | 3.14 | 3.44 | 3.62 | 3.21 | 3.48 | 3.49 |
| GSD | 2.04 | 2.01 | 1.96 | 2.00 | 2.00 | 1.96 |

**Table 5B. In vitro aerosolization performance formoterol emitted from MDI combination formulations of mometasone and formoterol in HFA-227ea and HFA-152a without oleic acid as characterised by the emitted dose, fine particle dose, fine particle fraction of the emitted dose (FPF_{ED}), mass median aerodynamic diameter (MMAD) and geometric standard deviation (GSD). These data are at Tzero, T = 1 Month @ 40°C/75 % RH valve down and T = 3 Months @ 40°C/75 % RH valve down.**

| | **HFA 227 - Tzero** | **HFA 227 - 1M@40/75** | **HFA 227 - 3M@40/75** | **152a - Tzero** | **152a - 1M@40/75** | **152a - 3M@40/75** |
|---|---|---|---|---|---|---|
| Emitted Dose (µg) | 4.7 ± 0.1 | 4.5 ± 0.1 | 4.2 ± 0.3 | 4.6 ± 0.2 | 4.9 ± 0.3 | 4.9 ± 0.3 |
| Fine Particle Dose (µg) | 1.3 ± 0.1 | 1.0 ± 0.1 | 0.8 ± 0.1 | 1.9 ± 0.1 | 2.1 ± 0.1 | 2.0 ± 0.2 |
| FPF_{ED} (%) | 27.7 | 22.2 | 19.0 | 41.3 | 42.9 | 40.8 |
| MMAD (µm) | 3.43 | 3.59 | 4.08 | 3.17 | 3.17 | 3.23 |
| GSD | 2.11 | 2.09 | 2.00 | 1.99 | 2.13 | 2.00 |

It can been seen from the tables above that when HFA-227ea was used as the propellant the omission of the oleic acid resulted in a substantial reduction in the emitted dose and the fine particle dose. In contrast, when HFA-152a was used as the propellant the aerosolization performance was similar with and without oleic acid. In addition, the formulations with HFA-152a were far more stable under the accelerated aging conditions with the formulations exhibiting better aerosolization performance than formulations with HFA-227ea, especially when oleic acid was omitted from the formulations.

### Example 2

The chemical stability of mometasone furoate and formoterol fumarate dihydrate in HFA-227ea and HFA-152a with and without oleic acid was investigated at time zero (T=0) and after storage, valve down, for 1 month (T=1M) and 3 months (T=3M) at 40°C and 75% relative humidity (RH) and at 25°C and 60% relative humidity (RH) in aluminium and steel cans.

The drug formulations were prepared as described in Example 1 above and analysed using the HPLC technique described in Example 1 above.

The results of investigating the chemical stability of the mometasone furoate (MMF) and formoterol fumarate dihydrate (FFD) combination drug formulations in HFA-152a and HFA-227ea are shown in Tables 6 to 9 below.

**Table 6. Chemical stability profile of mometasone (MMF)/formoterol (FFD) formulations in HFA-227ea with oleic acid at initial time-point, T = 1 Month and 3 Months at 25°C/60 % RH and 40°C /75 % RH.**

| **Time-Point** | **API** | **% Assay (LC)** | **% Total Imps (MMF+FFD)** |
|---|---|---|---|
| Initial Time Point | MMF | 99.2 | N.D. |
| | FFD | 99.5 | |
| T=1M@25/60 | MMF | 98.9 | 0.18 |
| | FFD | 97.5 | |
| T=1M@40/75 | MMF | 98.7 | 0.32 |
| | FFD | 98.3 | |
| T=3M@25/60 | MMF | 98.5 | 1.56 |
| | FFD | 97.6 | |
| T=3M@40/75 | MMF | 97.1 | 2.05 |
| | FFD | 95.4 | |

**Table 7. Chemical stability profile of mometasone (MMF)/formoterol (FFD) formulations in HFA-227ea without oleic acid at initial time-point, T = 1 Month and 3 Months at 25°C/60 % RH and 40°C /75 % RH.**

| **Time-Point** | **API** | **% Assay (LC)** | **% Total Imps (MMF+FFD)** |
|---|---|---|---|
| Initial Time Point | MMF | 99.8 | N.D. |
| | FFD | 99.2 | |
| T=1M@25/60 | MMF | 98.1 | 0.32 |
| | FFD | 97.4 | |
| T=1M@40/75 | MMF | 97.5 | 1.69 |
| | FFD | 96.4 | |
| T=3M@25/60 | MMF | 97.5 | 2.89 |
| | FFD | 92.6 | |
| T=3M@40/75 | MMF | 97.0 | 3.55 |
| | FFD | 91.8 | |

**Table 8. Chemical stability profile of mometasone (MMF)/formoterol (FFD) formulations in HFA-152a with oleic acid at initial time-point, T = 1 Month and 3 Months at 25°C/60 % RH and 40°C /75 % RH.**

| **Time-Point** | **API** | **% Assay (LC)** | **% Total Imps (MMF+FFD)** |
|---|---|---|---|
| Initial Time Point | MMF | 99.2 | N.D. |
| | FFD | 99.5 | |
| T=1M@25/60 | MMF | 99.9 | 0.09 |
| | FFD | 98.5 | |
| T=1M@40/75 | MMF | 99.2 | 0.15 |
| | FFD | 99.4 | |
| T=3M@25/60 | MMF | 98.3 | 0.48 |
| | FFD | 98.7 | |
| T=3M@40/75 | MMF | 98.5 | 0.55 |
| | FFD | 97.6 | |

**Table 9. Chemical stability profile of mometasone (MMF)/formoterol (FFD) formulations in HFA-152a without oleic acid at initial time-point, T = 1 Month and 3 Months at 25°C/60 % RH and 40°C /75 % RH.**

| **Time-Point** | **API** | **% Assay (LC)** | **% Total Imps (MMF+FFD)** |
|---|---|---|---|
| Initial Time Point | MMF | 99.8 | N.D. |
| | FFD | 99.9 | |
| T=1M@25/60 | MMF | 99.5 | N.D. |
| | FFD | 98.3 | |
| T=1M@40/75 | MMF | 99.5 | 0.11 |
| | FFD | 99.7 | |
| T=3M@25/60 | MMF | 99.1 | 0.32 |
| | FFD | 98.4 | |
| T=3M@40/75 | MMF | 98.3 | 0.49 |
| | FFD | 98.2 | |

It can be seen from the data in Tables 6 to 9 above that pharmaceutical formulations of mometasone furoate and formoterol fumarate dihydrate exhibit superior chemical stability when blended together with HFA-152a as the aerosolization propellant.

### Example 3

Formulations containing mometasone furoate and formoterol fumarate dihydrate and either HFA-227ea or HFA-152a were prepared in PET vials and the suspension stability of the formulations determined using a Turbiscan MA 2000. The Turbiscan instrument has a reading head that moves along a flat-bottomed, 5 mL cylindrical glass cell, and takes readings of transmitted and backscattered light every 40 µm on a maximum sample height of 80 mm. The reading head uses a pulsed near infrared light source and two synchronous detectors. The transmission detector picks up light transmitted through the suspension tube at 0° and back scattering detector receives light back by the product at 135°.

The sedimentation and size of flocs for the different formulations are shown in Table 10 below.

**Table 10. Suspension stability profiles of mometasone (MMF) and formoterol (FFD) in combination mometasone/formoterol formulations in HFA-227ea and HFA-152a with and without oleic acid (OA).**

| **Formulation** | **Size Start (microns)** | **Time to sediment (mins)** |
|---|---|---|
| MMF/FFD, Ethanol, OA and HFA-227ea | 3.82 | 1.85 |
| MMF/FFD, Ethanol, OA and HFA-152a | 3.42 | 3.55 |
| MMF/FFD, Ethanol and HFA-227ea | 5.89 | <0.5 |
| MMF/FFD, Ethanol and HFA-152a | 3.39 | 4.05 |

It can be seen from the data in Table 10 above that combination pharmaceutical formulations of mometasone furoate and formoterol fumarate dihydrate exhibit markedly superior settling performance when blended together with HFA-152a as the aerosolization propellant.

## Claims

1. A pharmaceutical composition comprising:
(i) at least one mometasone compound selected from mometasone and mometasone furoate;
(ii) formoterol fumarate dihydrate; and
(iii) a propellant component at least 90 weight % of which is 1,1-difluoroethane (R-152a).

2. The pharmaceutical composition of claim 1, wherein at least 95 weight %, preferably at least 98 weight % and more preferably at least 99 weight % of the composition consists of the three components (i), (ii) and (iii).

3. The pharmaceutical composition of claim 1 or 2, wherein the at least one mometasone compound consists entirely of mometasone furoate.

4. The pharmaceutical composition of any one of the preceding claims which after storage at 40°C and 75 % relative humidity for 3 months will produce less than 1.0 % by weight, preferably less than 0.8 % by weight, more preferably less than 0.7 % by weight and still more preferably less than 0.6 % by weight of impurities from the degradation of the at least one mometasone compound and the formoterol fumarate dihydrate based on the total weight of the at least one mometasone compound, the formoterol fumarate dihydrate and the impurities.

5. The pharmaceutical composition of any one of the preceding claims, wherein at least 97.0 % by weight and preferably at least 97.5 % by weight of the at least one mometasone compound and the formoterol fumarate dihydrate that are contained originally in the pharmaceutical composition immediately following preparation will be present in the composition after storage at 40°C and 75 % relative humidity for 3 months.

6. The pharmaceutical composition of any one of the preceding claims, which when delivered from a metered dose inhaler yields fine particle fractions of both the at least one mometasone compound and the formoterol fumarate dihydrate which are at least 30 weight % and preferably at least 35 weight % of the emitted doses of the at least one mometasone compound and the formoterol fumarate dihydrate even in the absence of a surfactant.

7. The pharmaceutical composition of any one of the preceding claims further comprising a surfactant component, preferably oleic acid.

8. The pharmaceutical composition of any one of claims 1 to 6 which is surfactant-free.

9. The pharmaceutical composition of any one of the preceding claims further comprising ethanol.

10. The pharmaceutical composition of any one of claims 1 to 8 which is free of ethanol.

11. The pharmaceutical composition of any one of the preceding claims, wherein at least 95 weight % and preferably at least 99 weight % of the propellant component is 1,1-difluoroethane (R-152a).

12. The pharmaceutical composition of claim 11, wherein the propellant component contains from 0.5 to 10 ppm, e.g. from 1 to 5 ppm, of unsaturated impurities.

13. The pharmaceutical composition of any one of the preceding claims, wherein the composition contains less than 100 ppm, preferably less than 50 ppm, more preferably less than 10 ppm and particularly less than 5 ppm of water based on the total weight of the pharmaceutical composition.

14. The pharmaceutical composition of claim 13, wherein the composition contains greater than 0.5 ppm, e.g. greater than 1 ppm, of water based on the total weight of the pharmaceutical composition.

15. The pharmaceutical composition of any one of the preceding claims which is free of one or both of perforated microstructures and acid stabilisers.

16. The pharmaceutical composition of any one of claims 1 to 14 which consists entirely of the three components (i), (ii) and (iii).

17. The pharmaceutical composition of any one of the preceding claims in the form of a suspension and wherein the drug particles in the suspension take at least 2.0 minutes, preferably at least 2.5 minutes and more preferably at least 3.0 minutes to settle following complete dispersion in the HFA-152a-containing propellant.

18. The pharmaceutical composition of claim 1, wherein at least 99 weight % of the composition comprises:
(i) a drug component consisting of mometasone furoate and formoterol fumarate dihydrate;
(ii) a propellant component at least 95 weight % of which is 1,1-difluoroethane (R-152a);
(iii) a surfactant component comprising oleic acid; and
(iv) ethanol.

19. A metered dose inhaler (MDI) fitted with a sealed and pressurised aerosol container that contains a pharmaceutical composition as claimed in any one of claims 1 to 18.

## Patentansprüche

1. Pharmazeutische Zusammensetzung, umfassend:
(i) mindestens eine Mometasonverbindung, ausgewählt aus Mometason und Mometasonfuroat;
(ii) Formoterolfumaratdihydrat; und
(iii) eine Treibmittelkomponente, wovon mindestens 90 Gew.-% 1,1-Difluorethan (R-152a) ist.

2. Pharmazeutische Zusammensetzung nach Anspruch 1, wobei mindestens 95 Gew.-%, vorzugsweise mindestens 98 Gew.-% und mehr bevorzugt mindestens 99 Gew.-%, der Zusammensetzung aus den drei Komponenten (i), (ii) und (iii) bestehen.

3. Pharmazeutische Zusammensetzung nach Anspruch 1 oder 2, wobei die mindestens eine Mometasonverbindung Mometasonfuroat ist.

4. Pharmazeutische Zusammensetzung nach einem der vorstehenden Ansprüche, die nach dreimonatiger Lagerung bei 40 °C und 75 % relativer Luftfeuchtigkeit weniger als 1,0 Gew.-%, vorzugsweise weniger als 0,8 Gew.-%, mehr bevorzugt weniger als 0,7 Gew.-% und noch mehr bevorzugt weniger als 0,6 Gew.-% Verunreinigungen aus dem Abbau der mindestens einen Mometasonverbindung und des Formoterolfumaratdihydrats, bezogen auf das Gesamtgewicht der mindestens einen Mometasonverbindung, des Formoterolfumaratdihydrats und der Verunreinigungen erzeugt.

5. Pharmazeutische Zusammensetzung nach einem der vorstehenden Ansprüche, wobei mindestens 97,0 Gew.-% und vorzugsweise mindestens 97,5 Gew.-% der mindestens einen Mometasonverbindung und des Formoterolfumaratdihydrats, die ursprünglich in der pharmazeutischen Zusammensetzung unmittelbar nach der Herstellung enthalten sind, nach dreimonatiger Lagerung bei 40 °C und 75 % relativer Luftfeuchtigkeit in der Zusammensetzung vorhanden sind.

6. Pharmazeutische Zusammensetzung nach einem der vorstehenden Ansprüche, die, wenn sie von einem Dosierinhalator abgegeben wird, feine Partikelfraktionen sowohl der mindestens einen Mometasonverbindung als auch des Formoterolfumaratdihydrats freigibt, die auch bei Abwesenheit eines Tensids mindestens 30 Gew.-% und vorzugsweise mindestens 35 Gew.-% der abgegebenen Dosen der mindestens einen Mometasonverbindung und des Formoterolfumaratdihydrats ausmachen.

7. Pharmazeutische Zusammensetzung nach einem der vorstehenden Ansprüche, ferner umfassend eine Tensidkomponente, vorzugsweise Ölsäure.

8. Pharmazeutische Zusammensetzung nach einem der Ansprüche 1 bis 6, die frei von Tensiden ist.

9. Pharmazeutische Zusammensetzung nach einem der vorstehenden Ansprüche, die ferner Ethanol umfasst.

10. Pharmazeutische Zusammensetzung nach einem der Ansprüche 1 bis 8, die frei von Ethanol ist.

11. Pharmazeutische Zusammensetzung nach einem der vorstehenden Ansprüche, wobei zu mindestens 95 Gew.-% und bevorzugt zu mindestens 99 Gew.-% die Treibmittelkomponente 1,1-Difluorethan (R-152a) ist.

12. Pharmazeutische Zusammensetzung nach Anspruch 11, wobei die Treibmittelkomponente von 0,5 bis 10 ppm, z. B. von 1 bis 5 ppm, ungesättigte Verunreinigungen enthält.

13. Pharmazeutische Zusammensetzung nach einem der vorstehenden Ansprüche, wobei die Zusammensetzung weniger als 100 ppm, vorzugsweise weniger als 50 ppm, mehr bevorzugt weniger als 10 ppm und insbesondere weniger als 5 ppm Wasser basierend auf dem Gesamtgewicht der pharmazeutischen Zusammensetzung enthält.

14. Pharmazeutische Zusammensetzung nach Anspruch 13, wobei die Zusammensetzung über 0,5 ppm, z. B. über 1 ppm, Wasser basierend auf dem Gesamtgewicht der pharmazeutischen Zusammensetzung enthält.

15. Pharmazeutische Zusammensetzung nach einem der vorstehenden Ansprüche, die frei von einer oder beiden perforierten Mikrostrukturen und Säurestabilisatoren ist.

16. Pharmazeutische Zusammensetzung nach einem der Ansprüche 1 bis 14, die vollständig aus den drei Komponenten (i), (ii) und (iii) besteht.

17. Pharmazeutische Zusammensetzung nach einem der vorstehenden Ansprüche in Form einer Suspension, und wobei die Arzneimittelpartikel in der Suspension mindestens 2,0 Minuten, vorzugsweise mindestens 2,5 Minuten und mehr bevorzugt mindestens 3,0 Minuten benötigen, um sich nach vollständiger Dispersion in dem HFA-152a enthaltenden Treibmittel abzusetzen.

18. Pharmazeutische Zusammensetzung nach Anspruch 1, wobei mindestens 99 Gew.-% der Zusammensetzung umfassen:
(i) eine Arzneimittelkomponente, zusammengesetzt aus Mometasonfuroat und Formoterolfumaratdihydrat;
(ii) eine Treibmittelkomponente, wovon mindestens 95 Gew.-% 1,1-Difluorethan (R-152a) sind;
(iii) eine Tensidkomponente, umfassend Ölsäure; und
(iv) Ethanol.

19. Dosierinhalator (MDI), der mit einem versiegelten und unter Druck stehenden Aerosolbehälter ausgerüstet ist, der eine pharmazeutische Zusammensetzung nach einem der Ansprüche 1 bis 18 enthält.

## Revendications

1. Composition pharmaceutique comprenant :
(i) au moins un composé de mométasone choisi parmi la mométasone et le furoate de mométasone ;
(ii) du formoterol fumarate dihydrate ; et
(iii) un composant propulseur dont au moins 90 % en poids est du 1,1-difluoroéthane (R-152a).

2. Composition pharmaceutique selon la revendication 1, dans laquelle au moins 95 % en poids, de préférence au moins 98 % en poids et plus préférablement au moins 99 % en poids de la composition est constitué des trois composants (i), (ii) et (iii).

3. Composition pharmaceutique selon la revendication 1 ou 2, dans laquelle l'au moins un composé de mométasone est entièrement constitué de furoate de mométasone.

4. Composition pharmaceutique selon l'une quelconque des revendications précédentes qui, après stockage à 40 °C et à 75 % d'humidité relative pendant 3 mois, produira moins de 1,0 % en poids, de préférence moins de 0,8 % en poids, plus préférablement moins de 0,7 % en poids et encore plus préférablement moins de 0,6 % en poids d'impuretés provenant de la dégradation de l'au moins un composé de mométasone et du formoterol fumarate dihydrate sur la base du poids total de l'au moins un composé de mométasone, du formoterol fumarate dihydrate et des impuretés.

5. Composition pharmaceutique selon l'une quelconque des revendications précédentes, dans laquelle au moins 97,0 % en poids et de préférence au moins 97,5 % en poids de l'au moins un composé de mométasone et du formoterol fumarate dihydrate qui sont contenus à l'origine dans la composition pharmaceutique immédiatement après la préparation seront présents dans la composition après stockage à 40 °C et à 75 % d'humidité relative pendant 3 mois.

6. Composition pharmaceutique selon l'une quelconque des revendications précédentes, qui, lorsqu'elle est administrée à partir d'un aérosol-doseur, donne de fines fractions de particules à la fois de l'au moins un composé de mométasone et du formoterol fumarate dihydrate qui sont au moins 30 % en poids et de préférence au moins 35 % en poids des doses émises de l'au moins un composé de mométasone et du formoterol fumarate dihydrate même en l'absence d'un tensioactif.

7. Composition pharmaceutique selon l'une quelconque des revendications précédentes, comprenant en outre un composant tensioactif, de préférence de l'acide oléique.

8. Composition pharmaceutique selon l'une quelconque des revendications 1 à 6 qui est exempte de tensioactif.

9. Composition pharmaceutique selon l'une quelconque des revendications précédentes, comprenant en outre de l'éthanol.

10. Composition pharmaceutique selon l'une quelconque des revendications 1 à 8 qui est exempte d'éthanol.

11. Composition pharmaceutique selon l'une quelconque des revendications précédentes, dans laquelle au moins 95 % en poids et de préférence au moins 99 % en poids du composant propulseur est le 1,1-difluoroéthane (R-152a).

12. Composition pharmaceutique selon la revendication 11, dans laquelle le composant propulseur contient de 0,5 à 10 ppm, par exemple de 1 à 5 ppm, d'impuretés insaturées.

13. Composition pharmaceutique selon l'une quelconque des revendications précédentes, dans laquelle la composition contient moins de 100 ppm, de préférence moins de 50 ppm, plus préférablement moins de 10 ppm et en particulier moins de 5 ppm d'eau sur la base du poids total de la composition pharmaceutique.

14. Composition pharmaceutique selon la revendication 13, dans laquelle la composition contient plus de 0,5 ppm, par exemple plus de 1 ppm, d'eau sur la base du poids total de la composition pharmaceutique.

15. Composition pharmaceutique selon l'une quelconque des revendications précédentes qui est exempte de l'un ou des deux parmi des microstructures perforées et des stabilisants acides.

16. Composition pharmaceutique selon l'une quelconque des revendications 1 à 14, qui est entièrement constituée des trois composants (i), (ii) et (iii).

17. Composition pharmaceutique selon l'une quelconque des revendications précédentes sous la forme d'une suspension et dans laquelle les particules de médicament dans la suspension prennent au moins 2,0 minutes, de préférence au moins 2,5 minutes et plus préférablement au moins 3,0 minutes pour se déposer après dispersion complète dans le propulseur contenant du HFA-152a.

18. Composition pharmaceutique selon la revendication 1, dans laquelle au moins 99 % en poids de la composition comprend :
(i) un composant médicamenteux constitué de furoate de mométasone et de formoterol fumarate dihydrate ;
(ii) un composant propulseur dont au moins 95 % en poids est du 1,1-difluoroéthane (R-152a) ;
(iii) un composant tensioactif comprenant de l'acide oléique ; et
(iv) de l'éthanol.

19. Aérosol-doseur (MDI) équipé d'un récipient aérosol étanche et sous pression qui contient une composition pharmaceutique selon l'une quelconque des revendications 1 à 18.
